# EUROPEAN PATENT SPECIFICATION

(11) **EP 0 480 762 B1**
(45) Date of publication and mention of the grant of the patent: **10.12.2003**
(21) Application number: 91309399.3
(22) Date of filing: 11.10.1991
(51) Int. Cl.: C12N 15/32, A01N 63/02, C12P 21/00

(54) **Novel bacillus thuringiensis isolate active against dipteran pests**
Gegen Diptere aktives, neues Bacillus-thuringiensis Isolat
Nouveau isolat de Bacillus thuringiensis actif contre les diptères

(30) Priority: 12.10.1990 US 596829; 29.01.1991 US 647399; 28.05.1991 US 708266; 21.08.1991 US 746751
(43) Date of publication of application: 15.04.1992
(60) Divisional application: 95107078.8
(73) Proprietor: MYCOGEN CORPORATION, San Diego, California 92121 (US)
(72) Inventor: Payne, Jewel M., San Diego, California 92126 (US); Uyeda, Kendrick Akira, San Diego, California 92109 (US); Stalder, Christine Julie, La Mesa, California 92041 (US); Michaels, Tracey Ellis, Escondido California 92027 (US)
(74) Representative: Perry, Robert Edward

(56) References cited:
- EP-A- 0 228 228
- EP-A- 0 409 438
- MICROBIOLOGICAL REVIEWS vol. 53, no. 2, June 1989, WASHINGTON DC, US pages 242 - 255 H\FTE, H. & WHITELEY, H.R. 'Insecticidal crystal proteins of Bacillus thuringiensis'
- BIOCHEMICAL AND BIOPHYSICAL RESEARCH COMMUNICATIONS vol. 137, no. 2, 13 June 1986, DULUTH, MINNESOTA US pages 748 - 751 SEKAR, V. 'Biochemical and immunological characterization of the cloned crystal toxin of Bacillus thuringiensis var. israelensis'
- JOURNAL OF BACTERIOLOGY vol. 170, no. 10, October 1988, AMERICAN SOCIETY FOR MICROBIOLOGY pages 4732 - 4738 DONOVAN, W.P. ET AL. 'Molecular characterization of a gene encoding a 72-kilodalton mosquito-toxic crystal protein from Bacillus thuringiensis subsp. israelensis'
- JOURNAL OF BACTERIOLOGY vol. 171, no. 6, 1989, pages 3060 - 3067 DROBNIEWSKI, F. A. ET AL. 'Purification and properties of a 28-kilodalton hemolytic and mosquitocidal protein toxin of Bacillus thuringiensis subsp. darmstadiensis 73-E10-2'

## Description

### Background of the Invention

Many hundreds of strains of Bacillus thuringiensis (B.t.) produce insecticidal toxins designated as delta endotoxins. They are synthesized by sporulating B.t. cells. When toxin is ingested by a susceptible insect, the cells of the gut epithelium are destroyed.

The reported activity spectrum of B.t. covers insect species within the orders Lepidoptera and Coleoptera, many of which are major pests in agriculture and forestry. The activity spectrum also includes the insect order Diptera, which includes mosquitoes and black flies. See Couch, T.L. (1980) "Mosquito Pathogenicity of Bacillus thuringiensis var. israelensis," Developments in Industrial Microbiology 22:61-76; Beegle, C.C. (1978) "Use of Entomogenous Bacteria in Agroecosystems," Developments in Industrial Microbiology 20:97-104. Dipteran insects are serious nuisances as well as being vectors of many serious human and animal diseases such as malaria, onchocerciasis, equine encephalitis, and dog heartworm.

The two varieties of B.t. known to kill mosquitos and blackflies are B.t. israelensis (B.t.i.) (Goldberg, L.J., J. Margalit [1977] Mosquito News 37:355-358) and B.t. morrisoni (B.t.m.) (Padua, L.E., M. Ohba, K. Aizawa [1984] J. Invertebrate Pathology 44:12-17). These are not harmful to non-target organisms (Mulla, M.S., B.A. Federici, H.A. Darwazeh [1982] Environmental Entomology 11:788-795), and play an important role in the integrated management of dipteran pests. They are safe to use in urban areas, and can be used in aquatic environments without harm to other species.

Dipteran pests are also a major problem in the poultry and cattle industries. The horn fly, a serious cattle pest, is killed by B.t. in the larval stages (Temeyer, K.B. [1990] "Potential of Bacillus thuringiensis for fly control," Fifth International Colloquium on Invertebrate Pathology and Microbial Control, Society for Invertebrate Pathology, 352-356). European Patent Application 90307204.9 (Publication No. 0 409 438) discloses Bacillus thuringiensis dipteran-active isolates PS71M3 and PS123D1.

Flies are an abundant species that can be found almost everywhere. They usually occur in such large numbers as to constitute a nuisance. The majority of the Diptera are considered pests and are of economic importance. A number of adult species are blood-sucking and cause irritation to man and his domestic animals. Others are scavenging flies that mechanically transmit organisms and pathogens that contaminate food. Both types of flies are important vectors of disease, such as malaria, yellow fever, filariasis, sleeping sickness, typhoid fever, and dysentery. Larvae of a few species are pests of major agriculture crops. The larvae can feed on all parts of the plant such as seeds, roots, leaves and fruits. Larvae of certain species feed on fungus causing damage to mushroom production. Larvae can irritate domestic animals when they develop in the animal. Both the adults and larvae are considered pests to man and agriculture.

House flies (family Muscidae) are an important pest from the order Diptera. They are considered a nuisance and are vectors of human and animal diseases. Their habits of walking and feeding on garbage and excrement and on the human person and food make them ideal agents for the transfer of disease organisms (Metcalf, C. and Flint, W. 1962. Destructive and Useful Insects, McGraw-Hill Book Co., NY, pp. 1030-1035.). This species can also be a pest to animals and transmit disease through open wounds. Muscidae also include the little house fly, face fly, stable fly, and horn fly, all of which are pests of livestock. These species are pests of cattle, poultry, horses and other types of livestock. They breed in manure and decaying straw located near the animals. The horn and stable flies are biting flies which cause stress to dairy cattle reducing milk production. The family Muscidae is considered an economic problem domestically and worldwide.

Leaf-mining flies cause damage and yield loss to economically important crops such as potatoes, tomatoes and celery. Dipteran leaf-miners are also considered a major pest in the ornamental flower industry (Parrella, M.P. 1987, Biology of Liriomyza, Ann. Rev. Entomol. 32:201-224). The most common leafminers are found in the family Agromyzidae although the families Anthomyiidae, Drosophilidae and Ephydridae also contain leaf-mining flies (Hespenheide, H.A. 1991, Bionomics of leafmining insects, Ann. Rev. Entomolo. 36:535-60). Flies in the genus Liriomyza (also known as serpentine leafminers) are particularly important because of their worldwide distribution, polyphagous nature and resistance to insecticides. In the state of California, the chrysanthemum industry lost approximately 93 million dollars to Liriomyza trifolii between the years of 1981-1985.

There are also dipteran pests of plants, such as Hessian fly, Medfly and Mexfly, for which a B.t. product would be very valuable.

### Summary of the Invention

According to the present invention, a novel Bacillus thuringiensis isolate identified herein as B.t. PS192N1, has activity against dipteran pests. The culture of the present invention was deposited in the Agricultural Research Service Patent Culture Collection (NRRL), Northern Regional Research Center, 1815 North University Street, Peoria, Illinois 61604, USA.

| **Culture** | **Accession No.** | **Deposit Date** |
|---|---|---|
| Bacillus thuringiensis PS192N1 | NRRL B-18721 | October S, 1990 |

The Bacillus thuringiensis isolate or toxins therefrom can be utilised as a spray for litter, manure, water, plant and other surfaces. They could also be used as feed through for domesticated animals and livestock. Transgenic plants and seeds could be used for control of stem, leaf and seed feeding maggots. Seeds could also be treated with a slurry of the isolate or toxin therefrom.

### Description of the Invention

The novel Bacillus thuringiensis isolate of the present invention has the following characteristics:
Colony morphology - large colony, dull surface, typical B.t.
Vegetative cell morphology - typical B.t.
Culture method - typical for B.t.
Flagellar serotype - 19, tochigiensis
Inclusion - amorphic
Alkali-soluble proteins - SDS polyacrylamide gels show 140, 122, 76, 72 and 38 kilodalton proteins
Insecticidal activity - B.t. PS192N1 has an LC₅₀ of 10 µg protein/ml suspension against Aedes aegypti

The novel B.t. isolate of the invention can be cultured using standard known media and fermentation techniques. Upon completion of the fermentation cycle, the bacteria can be harvested by first separating the B.t. spores and crystals from the fermentation broth means well known in the art. The recovered B.t. spores and crystals can be formulated into a wettable powder, a liquid concentrate, granules or other formulations by the addition of surfactants, dispersants, inert carriers and other components to facilitate handling and application for particular target pests. The formulation and application procedures are well known in the art and are used with commercial strains. The novel B.t. isolate can be used to control dipteran pests.

The toxin genes harbored by the novel isolates of the subject invention can be introduced into a wide variety of microbial hosts. Expression of the toxin gene results, directly or indirectly, in the intracellular production and maintenance of the pesticide. With suitable hosts, e.g., Pseudomonas, the microbes can be applied to the situs of dipteran insects where they will proliferate and be ingested by the insects. The result is a control of the unwanted insects. Alternatively, the microbe hosting the toxin gene can be treated under conditions that prolong the activity of the toxin produced in the cell. The treated cell then can be applied to the environment of target pest(s). The resulting product retains the toxicity of the B.t. toxin.

Where the B.t. toxin gene is introduced via a suitable vector into a microbial host, and said host is applied to the environment in a living state, it is essential that certain host microbes be used. Microorganism hosts are selected which are known to occupy the "'phytosphere" or aquatic environment. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves), and/or the rhizosphere (the soil surrounding plant roots), and/or aquatic environments. These microorganisms include bacteria, algae, and fungi. Of particular interest are microorganisms, such as bacteria, e.g., genera Bacillus, Pseudomonas, Erwinia, Serratia, Klebsiella, Xanthomonas, Streptomyces, Rhizobium, Rhodopseudomonas, Methylophilius, Agrobacterium, Acetobacter, Lactobacillus, Arthrobacter, Azotobacter, Leuconostoc, Acaligenes and Clostridium; fungi, particularly yeast, e.g., genera Saccharomyces, Cryptococcus, Kluyveromyces, Sporobolomyces, Rhodotorula, and Aureobasidium; microalgae, e.g., families Cyanophyceae, Prochlorophyceae, Rhodophyceae, Dinophyceae, Chrysophyceae, Prymnesiophyceae, Xanthophyceae, Raphidophyceae, Bacillariophyceae, Eustigmatophyceae, Cryptophyceae, Euglenophyceae, Prasinophyceae, and Chlorophyceae. Of particular interest are such phytosphere bacterial species as Pseudomonas syringae. Pseudomonas fluorescens, Serratia marcescens, Acetobacter xylinum, Agrobacterium tumefaciens, Rhodopseudomonas spheroides, Xanthomonas campestris, Rhizobium melioti, Alcaligenes entrophus, and Azotobacter vinlandii; and phytosphere yeast species such as Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae, and Aureobasidium pollulans. Of particular interest are the pigmented microorganisms.

A wide variety of ways are available for introducing a B.t. gene expressing a toxin into the microorganism host under conditions which allow for stable maintenance and expression of the gene. One can provide for DNA constructs which include the transcriptional and translational regulatory signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will only occur after release into the environment. This can be achieved with operators or a region binding to an activator or enhancers, which are capable of induction upon a change in the physical or chemical environment of the microorganisms. For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger RNA, particularly by using an active promoter, as well as by employing sequences, which enhance the stability of the messenger RNA. The transcriptional and translational termination region will involve stop codon(s), a terminator region, and optionally, a polyadenylation signal. A hydrophobic "leader" sequence may be employed at the amino terminus of the translated polypeptide sequence in order to promote secretion of the protein across the inner membrane.

In the direction of transcription, namely in the 5' to 3' direction of the coding or sense sequence, the construct will involve the transcriptional regulatory region, if any, and the promoter, where the regulatory region may be either 5' or 3' of the promoter, the ribosomal binding site, the initiation codon, the structural gene having an open reading frame in phase with the initiation codon, the stop codon(s), the polyadenylation signal sequence, if any, and the terminator region. This sequence as a double strand may be used by itself for transformation of a microorganism host, but will usually be included with a DNA sequence involving a marker, where the second DNA sequence may be joined to the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototropy to an auxotrophic host, or the like. Preferably, complementation is employed, so that the modified host may not only be selected, but may also be competitive in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin-producing host, so that it may effectively compete with the wild-type microorganisms and stably occupy a niche in the environment.

Where no functional replication system is present, the construct will also include a sequence of at least 50 basepairs (bp), preferably at least about 100 bp, and usually not more than about 5000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that a toxin gene is lost, the resulting organism will be likely to also lose the complementing gene and/or the gene providing for the competitive advantage, so that it will be unable to compete in the environment with the gene retaining the intact construct.

A large number of transcriptional regulatory regions are available from a wide variety of microorganism hosts, such as bacteria, bacteriophage, cyanobacteria, algae, fungi, and the like. Various transcriptional regulatory regions include the regions associated with the trp gene, lac gene, gal gene, the lambda left and right promoters, the tac promoter, the naturally-occurring promoters associated with the toxin gene, where functional in the host. See for example, U.S. Patent Nos. 4,332,898, 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host. The replication system may be derived from the chromosome, an episomal element normally present in the host or a different host, or a replication system from a virus which is stable in the host. A large number of plasmids are available, such as pBR322, pACYC184, RSF1010, pRO1614, and the like. See for example, Olson et al., (1982) J. Bacteriol. 150:6069, and Bagdasarian et al., (1981) Gene 16:237, and U.S. Patent Nos. 4,356,270, 4,362,817, and 4,371,625.

The B.t. gene can be introduced between the transcriptional and translational initiation region and the transcriptional and translational termination region, so as to be under the regulatory control of the initiation region. This construct will be included in a plasmid, which will include at least one replication system, but may include more than one, where one replication system is employed for cloning during the development of the plasmid and the second replication system is necessary for functioning in the ultimate host. In addition, one or more markers may be present, which have been described previously. Where integration is desired, the plasmid will desirably include a sequence homologous with the host genome.

The transformants can be isolated in accordance with conventional ways, usually employing a selection technique, which allows for selection of the desired organism as against unmodified organisms or transferring organisms, when present. The transformants then can be tested for pesticidal activity.

Suitable host cells, where the pesticide-containing cells will be treated to prolong the activity of the toxin in the cell when the then treated cell is applied to the environment of target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and - positive, include Enterobacteriaceae, such as Escherichia, Erwinia, Shigella, Salmonella, and Proteus; Bacillaceae; Rhizobiceae, such as Rhizobium; Spirillaceae, such as photobacterium, Zymomonas, Serratia, Aeromonas, Vibrio, Desulfovibrio, Spirillum; Lactobacillaceae; Pseudomonadaceae, such as Pseudomonas and Acetobacter; Azotobacteraceae, Actinomycetales, and Nitrobacteraceae. Among eukaryotes are fungi, such as Phycomycetes and Ascomycetes, which includes yeast, such as Saccharomyces and Schizosaccharomyces; and Basidiomycetes yeast, such as Rhodotorula, Aureobasidium, Sporobolomyces, and the like.

Characteristics of particular interest in selecting a host cell for purposes of production include ease of introducing the B.t. gene into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; survival in aqueous environments; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as Rhodotorula sp., Aureobasidium sp., Saccharomyces sp., and Sporobolomyces sp.; phylloplane organisms such as Pseudomonas sp., Erwinia sp. and Flavobacterium sp.; or such other organisms as Escherichia, Lactobacillus sp., Bacillus sp., Streptomyces sp., and the like. Specific organisms include Pseudomonas aeruginosa, Pseudomonas fluorescens, Saccharomyces cerevisiae, Bacillus thuringiensis, Escherichia coli, Bacillus subtilis, Streptomyces lividans and the like.

The cell will usually be intact and be substantially in the proliferative form when treated, rather than in a spore form, although in some instances spores may be employed.

Treatment of the microbial cell, e.g., a microbe containing the B.t. toxin gene, can be by chemical or physical means, or by a combination of chemical and/or physical means, so long as the technique does not deleteriously affect the properties of the toxin, nor diminish the cellular capability in protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride and cetylpyridinium chloride; alcohols, such as isopropyl and ethanol; various histologic fixatives, such as Lugol iodine, Bouin's fixative, and Helly's fixative (See: Humason, Gretchen L., Animal Tissue Techniques, W.H. Freeman and Company, 1967); or a combination of physical (heat) and chemical agents that preserve and prolong the activity of the toxin produced in the cell when the cell is administered to the host animal. Examples of physical means are short wavelength radiation such as gamma-radiation and X-radiation, freezing, UV irradiation, lyophilization, and the like.

The cells generally will have enhanced structural stability which will enhance resistance to environmental conditions. Where the pesticide is in a proform, the method of inactivation should be selected so as not to inhibit processing of the proform to the mature form of the pesticide by the target pest pathogen. For example, formaldehyde will crosslink proteins and could inhibit processing of the proform of a polypeptide pesticide. The method of inactivation or killing retains at least a substantial portion of the bio-availability or bioactivity of the toxin.

The cellular host containing the B.t. insecticidal gene may be grown in any convenient nutrient medium, where the DNA construct provides a selective advantage, providing for a selective medium so that substantially all or all of the cells retain the B.t. gene. These cells may then be harvested in accordance with conventional ways. Alternatively, the cells can be treated prior to harvesting.

The B.t. cells may be formulated in a variety of ways. They may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about 10² to about 10⁴ cells/mg. These formulations will be administered at about 50 mg (liquid or dry) to 1 kg or more per hectare.

The formulations can be applied to the environment of the dipteran pest(s), e.g., plants, soil or water, by spraying, dusting, sprinkling, or the like.

Mutants of the novel isolates of the invention can be made by procedures well known in the art. For example, an asporogenous mutant can be obtained through ethylmethane sulfonate (EMS) mutagenesis of a novel isolate. The mutants can be made using ultraviolet light and nitrosoguanidine by procedures well known in the art.

A smaller percentage of the asporogenous mutants will remain intact and not lyse for extended fermentation periods; these strains are designated lysis minus (-). Lysis minus strains can be identified by screening asporogenous mutants in shake flask media and selecting those mutants that are still intact and contain toxin crystals at the end of the fermentation. Lysis minus strains are suitable for a cell fixation process that will yield a protected, encapsulated toxin protein.

To prepare a phage resistant variant of said asporogenous mutant, an aliquot of the phage lysate is spread onto nutrient agar and allowed to dry. An aliquot of the phage sensitive bacterial strain is then plated directly over the dried lysate and allowed to dry. The plates are incubated at 30°C. The plates are incubated for 2 days and, at that time, numerous colonies could be seen growing on the agar. Some of these colonies are picked and subcultured onto nutrient agar plates. These apparent resistant cultures are tested for resistance by cross streaking with the phage lysate. A line of the phage lysate is streaked on the plate and allowed to dry. The presumptive resistant cultures are then streaked across the phage line. Resistant bacterial cultures show no lysis anywhere in the streak across the phage line after overnight incubation at 30°C. The resistance to phage is then reconfirmed by plating a lawn of the resistant culture onto a nutrient agar plate. The sensitive strain is also plated in the same manner to serve as the positive control. After drying, a drop of the phage lysate is plated in the center of the plate and allowed to dry. Resistant cultures showed no lysis in the area where the phage lysate has been placed after incubation at 30°C for 24 hours.

Following are examples which illustrate procedures, including the best mode, for practicing the invention. These examples should not be construed as limiting. All percentages are by weight and all solvent mixture proportions are by volume unless otherwise noted.

### Example 1 - Culturing of the Novel B.t. Isolates

A subculture of the novel B.t. isolates, or mutants thereof, can be used to inoculate the following medium, a peptone, glucose, salts medium.

| | |
|---|---|
| Bacto Peptone | 7.5 g/l |
| Glucose | 1.0 g/l |
| KH₂PO₄ | 3.4 g/l |
| K₂HPO₄ | 4.35 g/l |
| Salt Solution | 5.0 ml/l |
| CaCl₂ Solution | 5.0 ml/l |

| Salts Solution (100 ml) | |
|---|---|
| MgSO₄.7H₂O | 2.46 g |
| MnSO₄.H₂O | 0.04 g |
| ZnSO₄.7H₂O | 0.28 g |
| FeSO₄.7H₂O | 0.40 g |

| CaCl₂ Solution (100 ml) | |
|---|---|
| CaCl₂.2H₂O | 3.66 g |
| pH 7.2 | |

The salts solution and CaCl₂ solution are filter-sterilized and added to the autoclaved and cooked broth at the time of inoculation. Flasks are incubated at 30°C on a rotary shaker at 200 rpm for 64 hr.

The above procedure can be readily scaled up to large fermentors by procedures well known in the art.

The B.t. spores and/or crystals, obtained in the above fermentation, can be isolated by procedures well known in the art. A frequently-used procedure is to subject the harvested fermentation broth to separation techniques, e.g., centrifugation.

### Example 2 - Purification of protein

The B. thuringiensis isolate was cultured as described in Example 1. the parasporal inclusion bodies (toxin crystals) were partially purified by sodium bromide (26-40%) isopycnic gradient centrifugation (Pfannestiel, M.A., EJ. Ross, V.C. Kramer, and K.W. Nickerson [1984] FEMS Microbiol. Lett. 21:39).

### Example 3 - Activity Against Aedes aegypti

Aedes aegypti the yellow fever mosquito, is used as an indicator of mosquito activity. The bioassay is performed on a spore and crystal suspension or a suspension of purified crystals. Dilutions of the suspension are added to water in a small cup. Third instar larvae are added, and mortality is read after 48 hours.

B.t. PS192N1 has an LC₅₀ of 10 µm protein/ml suspension against Aedes aegypti.

## Claims

1. A culture of a microbe of the species *Bacillus thuringiensis* identified as PS192N1, having the NRRL accession number B-18721.

2. A composition comprising a microbe as defined in claim 1, in association with an insecticide carrier.

3. A composition comprising a stable lysis⁻spo⁻cry⁺ *Bacillus thuringiensis* microbe as prepared from a microbe as defined in claim 1, in association with an insecticide carrier.

4. A method for controlling dipteran insect pests, which comprises contacting the pests with a microbe as defined in claim 1, or spores thereof or a toxin obtainable therefrom.

5. A method according to claim 4, wherein the pests are mosquitoes.

6. A method according to claim 4 or claim 5, wherein the pests are contacted with intact, treated cells containing the toxin intracellularly.

## Patentansprüche

1. Kultur einer Mikrobe der Art *Bacillus thuringiensis*, identifiziert als PS192N1, mit der NRRL-Hinterlegungsnummer B-18721.

2. Zusammensetzung, umfassend eine Mikrobe, wie in Anspruch 1 definiert, in Verbindung mit einem Insektizid-Träger.

3. Zusammensetzung, umfassend eine stabile Lysis⁻spo⁻cry⁺ *Bacillus thuringiensis*-Mikrobe, hergestellt aus einer in Anspruch 1 definierten Mikrobe, in Verbindung mit einem Insektizid-Träger.

4. Verfahren zur Kontrolle von zweiflügligen Insektenschädlingen, welches umfasst, dass man die Schädlinge mit einer Mikrobe, wie in Anspruch 1 definiert, oder Sporen derselben oder einem Toxin, das aus derselben erhältlich ist, in Kontakt bringt.

5. Verfahren nach Anspruch 4, in dem die Schädlinge Moskitos sind.

6. Verfahren nach Anspruch 4 oder Anspruch 5, in dem die Schädlinge mit intakten, behandelten Zellen, die das Toxin intrazellulär enthalten, in Kontakt gebracht werden.

## Revendications

1. Culture d'un microbe de l'espèce *Bacillus thuringiensis* identifié sous le nom de PS192N1, ayant le numéro d'accession NRRL B-18721.

2. Composition comprenant un microbe tel que défini dans la revendication 1, associé à un véhicule insecticide.

3. Composition comprenant des spores et cristaux d'un microbe de Bacillus thuringiensis stable à la lyse tel que préparée à partir d'un microbe défini dans la revendication 1, associé à un véhicule insecticide.

4. Procédé de contrôle d'insectes diptères nuisibles, qui comprend la mise en contact des insectes nuisibles avec un microbe tel que défini dans la revendication 1, ou des spores de celui-ci ou une toxine que l'on peut obtenir de celui-ci.

5. Procédé selon la revendication 4, dans lequel les insectes nuisibles sont des moustiques.

6. Procédé selon la revendication 4 ou 5, dans lequel les insectes nuisibles sont mis au contact de cellules traitées intactes contenant de la toxine intracellulaire.
